Europäisches Patentamt

European Patent Office

Office européen des brevets

(11) Publication number: **0 336 742**
**A2**

## EUROPEAN PATENT APPLICATION

(21) Application number: **89303381.1**

(22) Date of filing: **05.04.89**

(51) Int. Cl.⁴: **A 61 B 17/36**

(30) Priority: **08.04.88 US 179401**

(43) Date of publication of application:
**11.10.89 Bulletin 89/41**

(84) Designated Contracting States: **DE FR GB IT**

(71) Applicant: **Bristol-Myers Company**
**345 Park Avenue**
**New York New York 10154 (US)**

(72) Inventor: **Logsdon, Edward Louis**
**14130 E. Temple Drive**
**Aurora Colorado (US)**

**Gans, Anthony Richard**
**34 Pine Road**
**Golden Colorado 80439 (US)**

(74) Representative: **Foster, David Martyn et al**
**MATHISEN MACARA & CO. The Coach House 6-8**
**Swakeleys Road**
**Ickenham Uxbridge UB10 8BZ (GB)**

(54) Method and apparatus for the calibration of electrosurgical apparatus.

(57) A method and apparatus for the calibration of electrosurgical apparatus utilizes a non-volatile, read-write storage device (U2) to store calibration parameters. A programmed microprocessor (U3) and an EPROM memory (U1) for the microprocessor (U3) control the flow of data to and from the storage device (U2). For the calibration of the RF power output a test resistor of known value provides a test power reading and the RF power output is adjusted by the adjustment of up/down power select keys until the RF power output substantially equals the test power so that the values stored in the storage device (U2) control subsequent RF power output levels. For the calibration of a return electrode contact monitor (35), a low resistance of known value is input into the storage device (U2) by depressing a key and a high resistance value is input into the storage device (U2) by depressing the same key. These values are stored in the storage device (U2) and provide a transfer function from which a particular output voltage value is generated for each return electrode resistance measurement.

FIG.1

**Description**

## METHOD AND APPARATUS FOR THE CALIBRATION OF ELECTROSURGICAL APPARATUS

The invention relates to electrosurgical apparatus and more particularly to a method and apparatus for the calibration of electrosurgical apparatus.

In the past, the calibration of electrosurgical apparatus has been accomplished by making manual hardware-type adjustments, such as the adjusting the setting of potentiometers or related components with a screw driver or the like. The disadvantage of this approach is the many components must be adjusted and such calibration is time consuming and costly.

US-PS 4 716 897 discloses electrosurgical apparatus wherein an internal dummy load is connected to internal terminals for an internal measurement. A measured error or estimated correction value is used in an open-loop type system which is compared with a setting value to correct the RF signal. This is essentially an estimated correction. The RF output level is controlled on the basis of data stored in an EPROM using a PROM writer. Once information is written into the EPROM the information must be erased physically or by non-electrical means which requires considerable time and is relatively difficult.

According to the invention, there is provided a method of calibrating selected operating characteristics of electrosurgical apparatus, comprising the step of storing selected calibration parameters establishing corresponding selected operating characteristics of electrosurgical apparatus, characterised in that the parameters are stored in a non-volatile, read-write storage device capable of being altered in place by electrical means for controlling of the subsequent operation of said electrosurgical apparatus.

According to the invention, there is also provided apparatus for calibrating selected operating characteristics of electrosurgical apparatus comprising storage means for storing selected calibration parameters establishing corresponding operating characteristics in electrosurgical apparatus, characterised in that the storage means is a non-volatile, read-write storage device capable of being altered in place by electrical means for controlling the operation of said electrosurgical apparatus.

According to the invention, there is further provided apparatus for calibrating the RF power output of electrosurgical apparatus including a display for indicating RF power output and up/down select keys for changing the RF power output and a RF amplifier providing the RF power output that is coupled to a load resistor of a known value and providing a display of test power, characterised by a controllable signal generating means responsive to the setting of said select keys and coupled to said RF amplifier to regulate the power output, a non-volatile, read-write storage device for storing calibration drive variables according to the operation of the up/down select keys whereby upon the movement of the select keys until the power output equals the test power the power output is calibrated, and a programmed microprocessor coupled to a memory and to said storage device for controlling said signal generating means according to the drive variable stored in said storage device.

According to the invention, there is yet further provided apparatus for calibrating the RF power output of electrosurgical apparatus coupled to a test load having a visual display of power consumed by a load resistance of known value, characterised by a programmed microprocessor, a memory coupled to said microprocessor providing the processing instructions for the microprocessor and storing initial desired power values and corresponding drive variable values, a waveform generator coupled to said microprocessor and to an RF amplifier for providing a waveform from the RF amplifier of a selected duty cycle, a digital to analog converter coupled to said microprocessor and to the RF amplifier for controlling the amplitude of the waveform produced by the waveform generator, a non-volatile, read-write storage device coupled to said microprocessor for storing and outputting to the microprocessor the calibrated RF power output values and corresponding drive variable values after the RF power output has been calibrated, a display for indicating actual RF power output that is coupled to the microprocessor, said display initially indicating a calibration point number in amps corresponding to actual RF power being output, up/down keys on the display for changing the RF power output, said microprocessor receiving a test power output value corresponding to said calibration point number from said storage device, said microprocessor driving said non-volatile, read-write storage device with said test power value and retrieving a drive variable value from a table stored therein, said microprocessor providing an output to said converter or waveform generator which in turn causes said RF amplifier to output a corresponding RF power output, said up/down keys being adjusted until the display power substantially equals the power reading in the test load, said microprocessor adjusting the power value in the storage device according to the new setting to store a new power output value and corresponding drive variable value thereby calibrating the apparatus to deliver the new RF power output during subsequent operations.

Apparatus embodying the invention, and methods according to the invention, for the calibration of electrosurgical apparatus will now be described, by way of example only, with reference to the accompanying drawings in which:-

Figure 1 is a schematic diagram of the apparatus;

Figure 2 is a schematic diagram of the apparatus shown in Figure 1 and coupled to a test box for the calibration of RF power output;

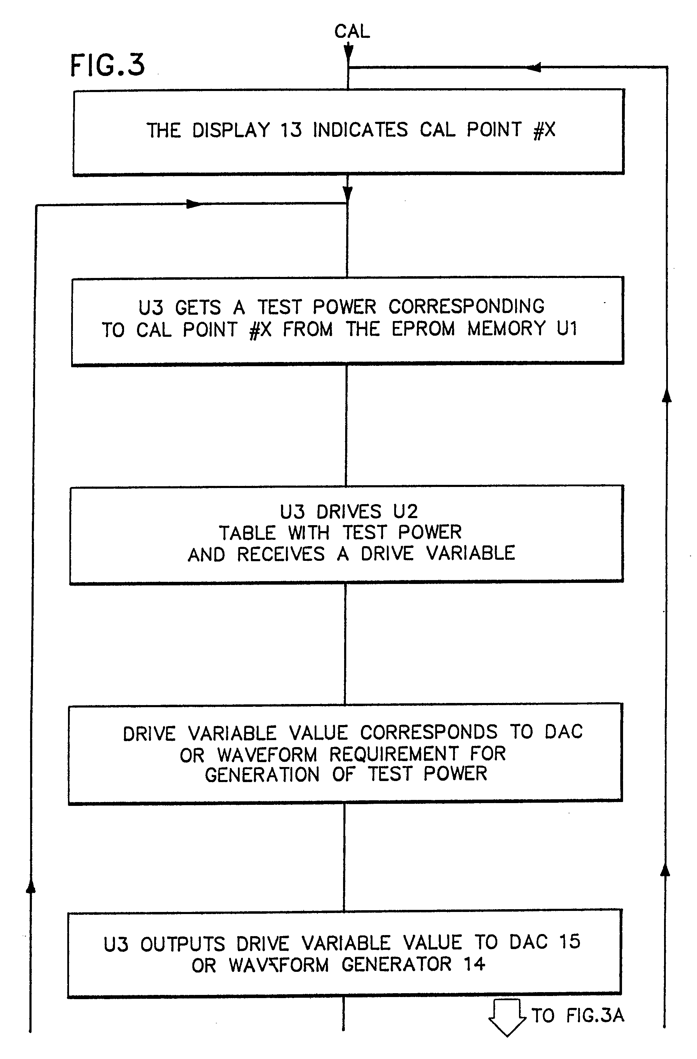

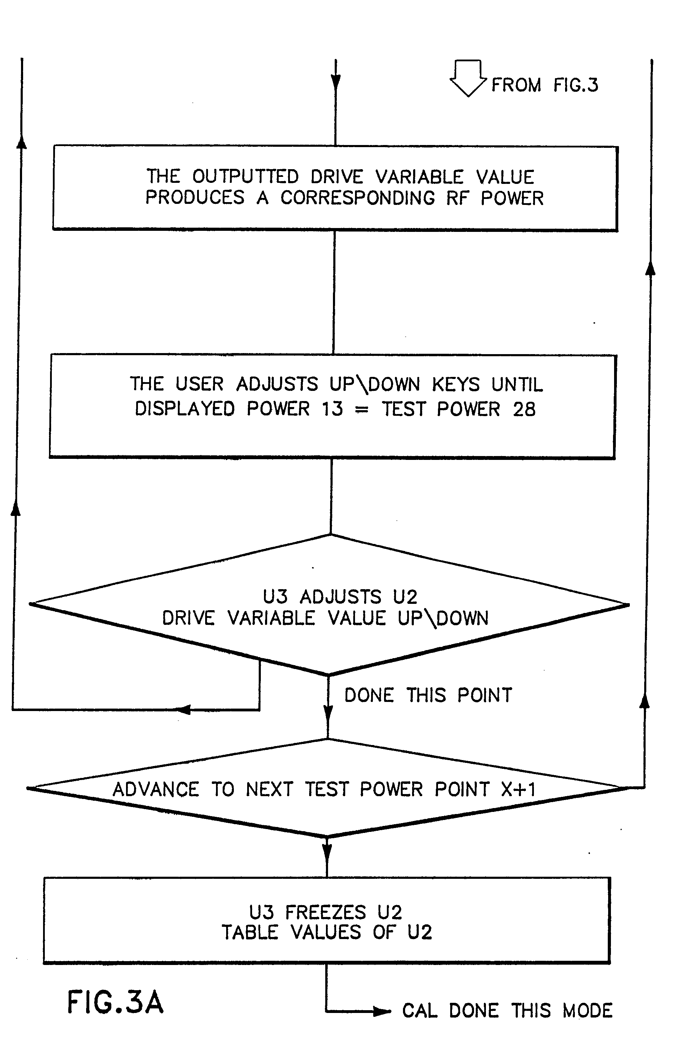

Figures 3 and 3A are a flow chart of the program for a microprocessor in the apparatus of Figure 1 for the calibration of RF power output;

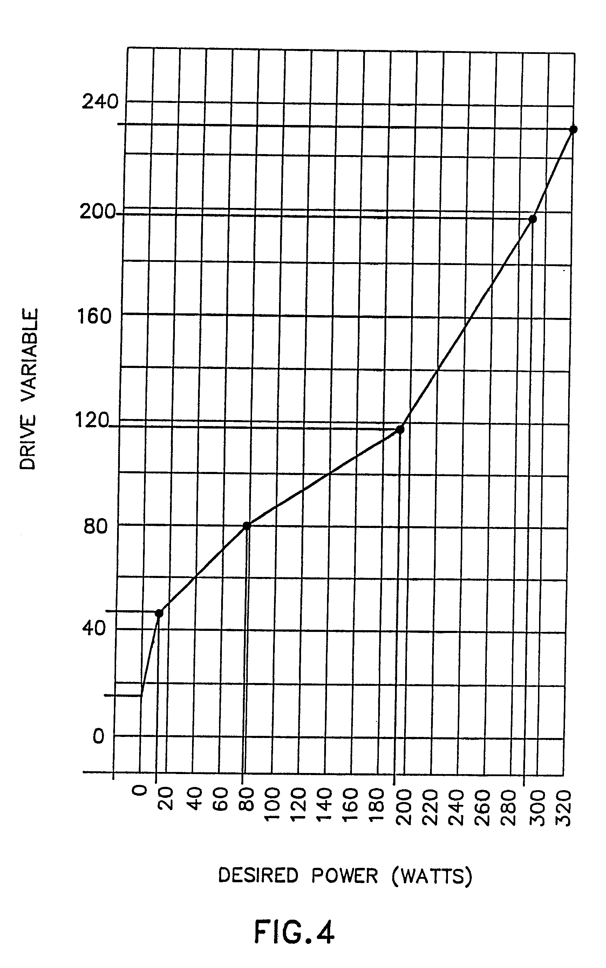

Figure 4 is a graph showing five points on a plot of desired power and drive variable values for the calibration of RF power output in the apparatus;

Figure 5 is a schematic diagram illustrating the function performed in the software to convert from power to a drive variable value according to the table values in the non-volatile, read-write storage device in the apparatus;

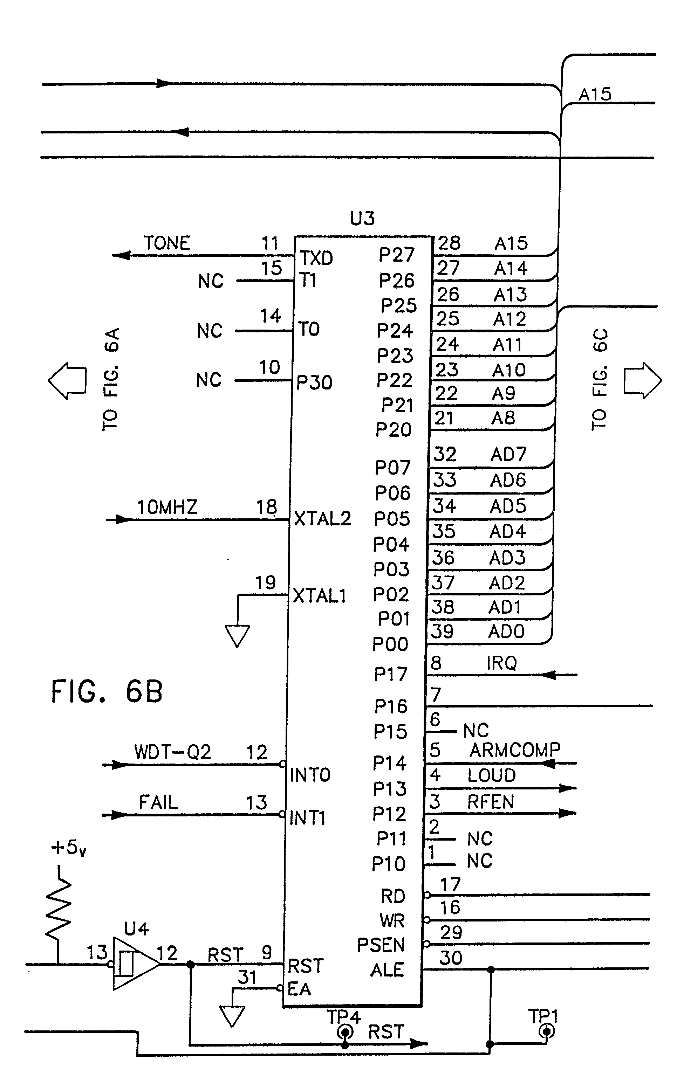

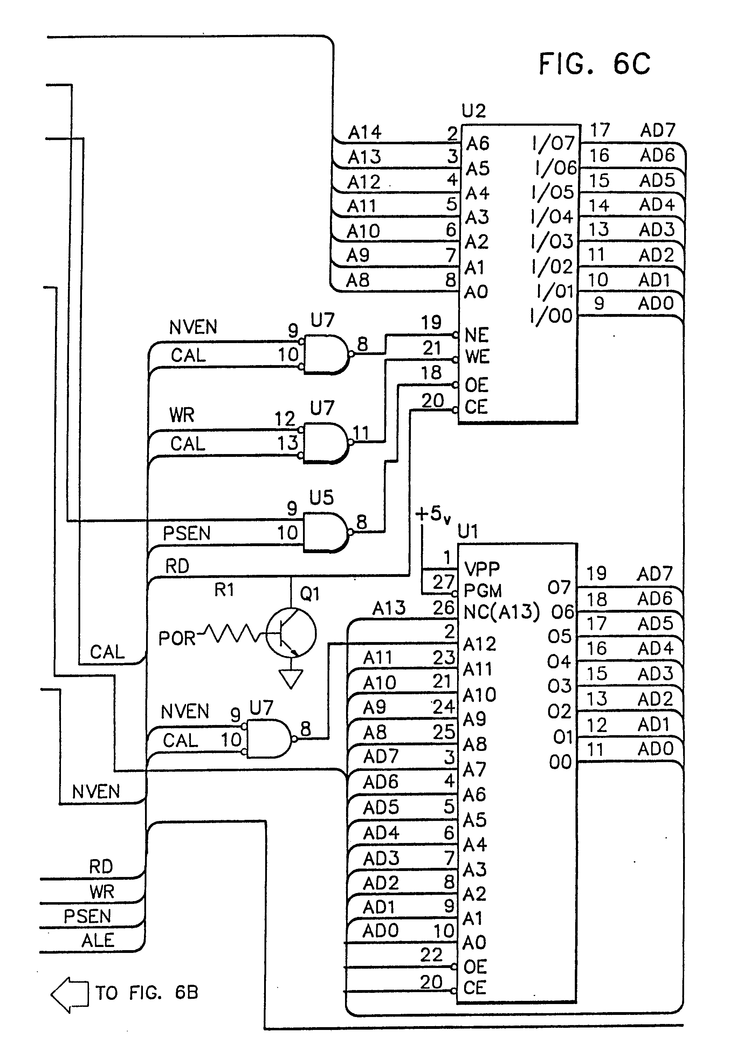

Figure 6A, 6B, and 6C are more detailed circuit diagrams of the apparatus shown in Figure 1;

Figure 7 is a schematic diagram of the apparatus as coupled to a resistor for calibrating a return electrode contact monitor;

Figure 8 is a more detailed schematic diagram of the apparatus for calibrating the return electrode contact monitor;

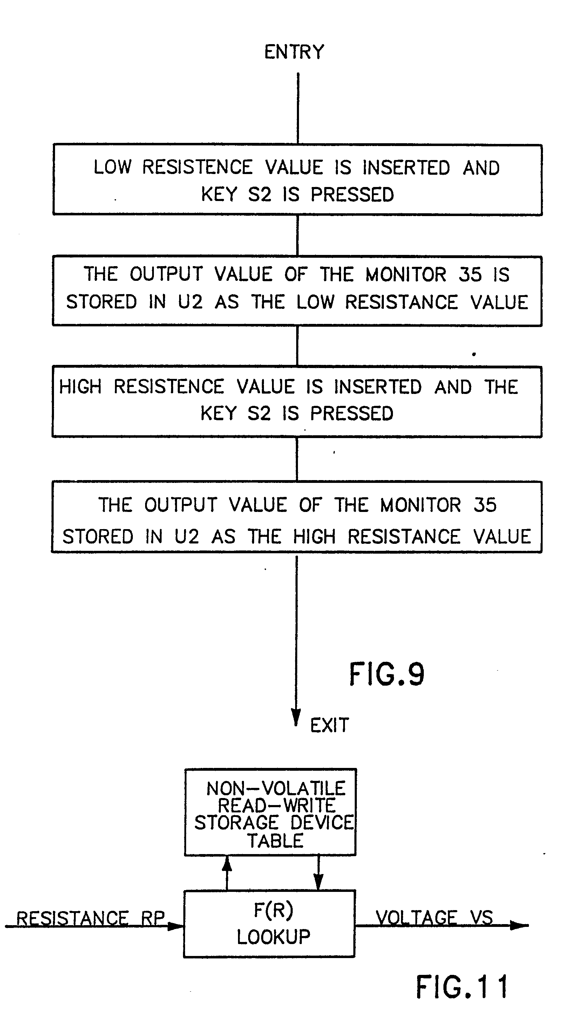

Figure 9 is a flow chart of the program for the microprocessor for accomplishing the calibration of the return electrode contact monitor;

Figure 10 is a graph showing the relationship of resistor and the output voltage VS produced by the return electrode contact monitor for selected low and high resistance values for the calibration of the return electrode contact monitor; and

Figure 11 is a schematic diagram illustrating the function performed in the software to convert from resistance to voltage according to table values in the non-volatile, read-write storage device.

The apparatus to be described uses a non-volatile read-write storage device to store calibration parameters. A programmed microprocessor and an EPROM memory providing processing instructions for the microprocessor serve to control date flow to and from the storage device. Calibration of two illustrative operating characteristics disclosed are the RF power output and the return electrode contact monitor. For calibrating RF power output the storage device stores a tabular group of number values corresponding to a range of power (watts) in selected watt increments and these are drive variable values that are modified in the calibration procedure. A load resistor of known value is connected to RF power output terminals giving a test power value. The RF power output is adjusted by up/down select keys until the RF power output is substantially equal to the test power which results in a change in the drive variable value stored in the storage device to establish the actual RF power output during the next surgical operation. For calibrating the return electrode contact monitor, a selected low resistance value is connected to the monitor and an alarm set point key is pushed, followed by connecting a selected high resistance value to the monitor and again pressing the alarm set point key. These two resistance values are stored in the storage device to calibrate the output voltage for each resistance measured by the monitor.

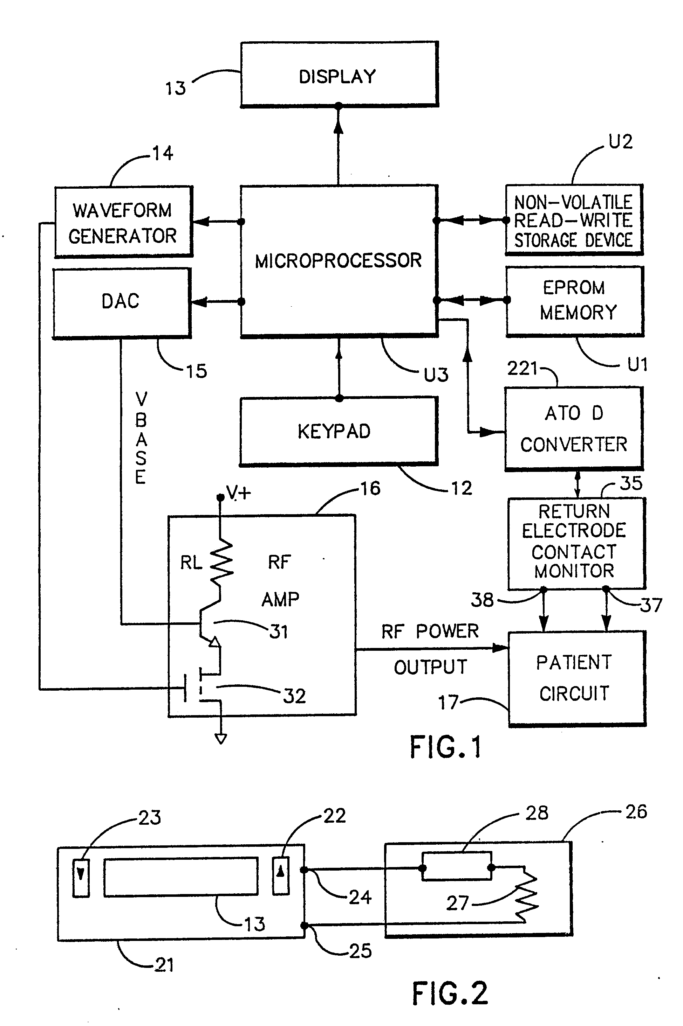

Referring now in more detail to Figure 1, there is shown calibration apparatus which includes non-volatile read-write storage device U2 that is operatively coupled to a programmed microprocessor U3 which in turn is operatively coupled to an EPROM memory U1. A key pad I2 and a display 13 are operatively coupled to microprocessor U3.

The non-volatile read-write storage device U2 which may also be referred to as a non-volatile random access memory has advantages over devices such as the EPROM disclosed by the above discussed US-PS 4 716 897. The EPROM is a read-only memory and requires a non-electrical or a physical erasure such as by removing the element and placing it under ultra-violet light. The storage device U2 is a read-write memory which is quickly and easily reconfigured by electrical means. A non-volatile storage device is one that can retain information in the absence of power. The apparatus being described uses the same routines in both the calibration and the run modes of operation without any reconfiguration.

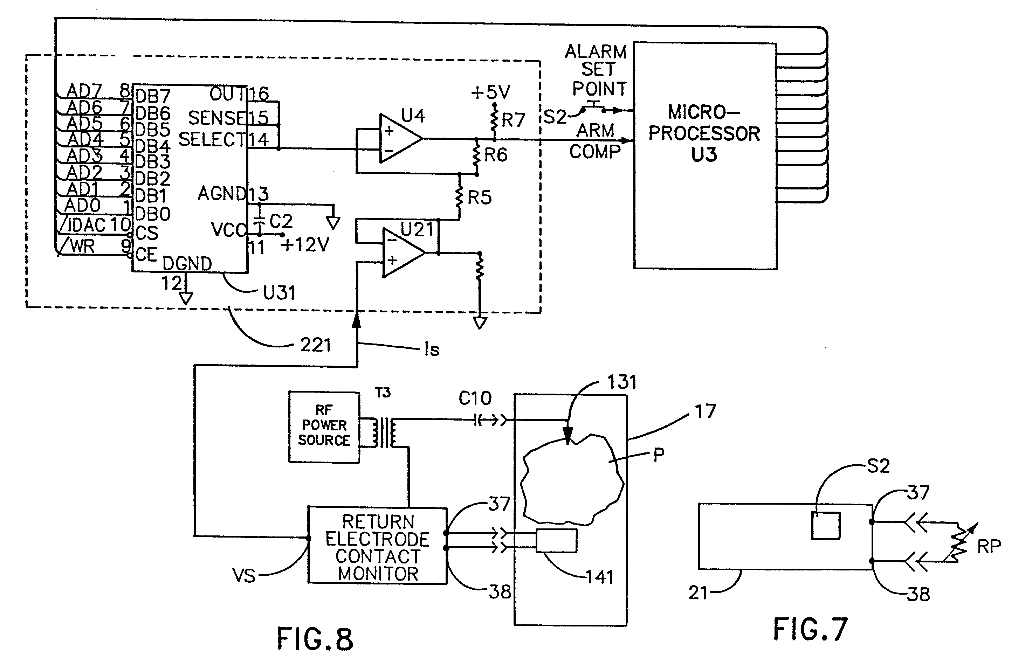

The apparatus is suitable for calibrating different operating characteristics of the electrosurgical apparatus. One operating characteristic that can be calibrated is the RF power output. In connection with controlling the RF power output there is provided a controllable signal generating means which includes waveform generator 14 and a digital to analog converter (DAC) 15 which generates a VBASE signal. Elements I4 and 15 receive outputs from the microprocessor U3 with each being coupled into a RF amplifier 16 providing the RF power output for the electrosurgical apparatus. The RF amplifier 16 is shown coupled to a patient circuit 17 which includes the usual active electrode 131 and return electrode 141 (Figure 8).

In Figure 2 there is shown a block 21 depicting a housing containing the electrosurgical apparatus. The housing has a front panel display 13, which displays actual power put out by the apparatus with an up key 22 and a down key 23 for adjusting the RF power output either up or down, respectively, as required. The RF power output appears at output terminals 24 and 25 of the electrosurgical apparatus which are shown as coupled to a test box 26 having a load resistor 27 of a known value and a power meter 28 indicating amps. Meter 28 provides a measure of the actual power consumption of load 27 which is herein referred to as test power.

The RF power output of the electrosurgical apparatus 21 is controlled by the signal generator means depending on the mode selected. The output of the DAC 15 (V BASE signal) is applied to the base of a transistor 31 (Fig. 1). The output of the waveform generator is applied to an electrode of a MOSFET 32. The emitter and collection electrodes of transistor 31 and the electrodes of MOSFET 32 are connected in a series circuit including load resistor RL between a bias voltage V+ and ground. In one mode the duty cycle of the waveform generator 14 is varied with a fixed bias voltage. Another mode is to vary the bias voltage with a fixed duty cycle for the output waveform which varies the amplitude of the waveform. In yet a third mode both the duty cycle and the bias voltage may be varied. These modes involve the generation of a drive variable value (0-255) with a subsequent loading of the drive variable to control the DAC 15 and the waveform generator 14. The calibration apparatus includes the non-volatile, read-write storage device U2 which stores a table or tabular group of 17 number values per mode corresponding to 0-192 output watts in 16 watt increments or 192-320 output watts in 32 watt increments. Values required between the 17 table entries are found by linear interpolation. These values are stored in the non-volatile, read-write storage device U2 and are modified by the calibration procedure.

The calibration procedure for the calibration of RF power output involves connecting the RF power output terminals 24 and 25 to a load resistor 27 of a known value and raising or lowering the test power observed on the meter 28 until it is substantially the same as the value displayed on the front panel display 13. Internally, the apparatus is raising or lowering the drive variable value in the non-volatile, read-write storage device U2 which in turn controls the output power for each mode. When a calibration has been completed new drive variable values generated during the calibration procedure are permanently stored in the non-volatile, read-write storage device U2 for controlling the power output for the next surgical procedure.

Another operating characteristic that is calibrated in the apparatus being described is the return electrode contact monitor 35. The return electrode contact monitor 35 is described in more detail in copending European Patent Application No. 89301514.9. Generally stated, this monitor measures the quality of the contact between the return electrode 141 and the patient P (Figure 8). In particular, the monitor 35 measures the resistance of the return part of the patient circuit 17 at measurement terminals 37 and 38. Terminals 37 and 38 are normally connected to the return electrode 141 in the patient circuit 17. As described in detail in the above mentioned copending application, the monitor 35 when coupled to the return electrode produces a DC voltage VS that varies in relation to the resistance of the return part of the patient circuit. An analog to digital converter 221 converts VS to a corresponding binary digital signal that is delivered to microprocessor U3.

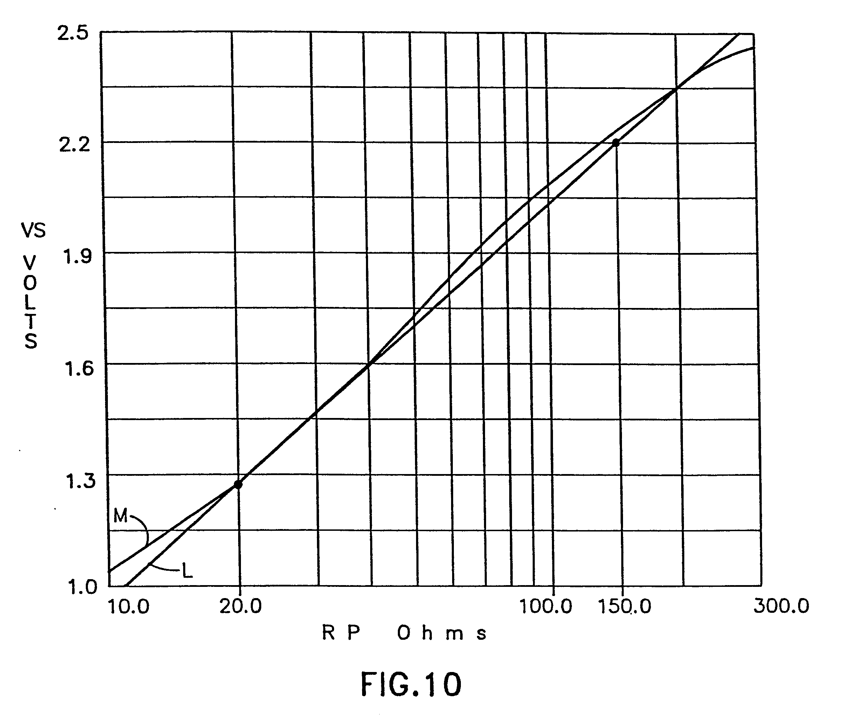

The return electrode contact monitor 35 is calibrated using two resistances of a selected known value herein referred to as a low resistance value and a high resistance value. As shown in Figure 10 the low resistance value is 20 ohms and the high resistance value is 150 ohms.

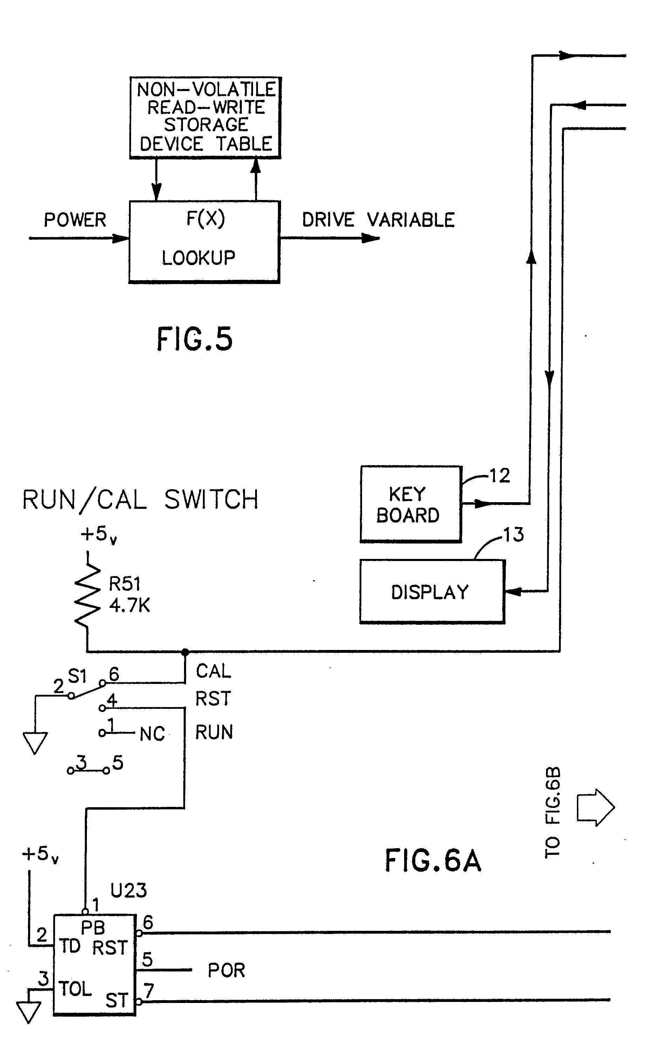

In the calibration procedure for the monitor 35 the low resistance value RP (20 ohms) is connected across terminals 37 and 38 and the alarm set point key (switch) S2 is pushed. After the low resistance value is removed the high resistance value 150 ohms is connected across terminals 37 and 38 and the alarm set point key S2 is pushed. This stores two resistance values or points in the storage device U2 like the values stored during the calibration of the RF power output as above described. A functional block similar to Figure 5 is shown in Figure 11 for the calibration procedure for the monitor. Referring now to Figure 10 after the calibration procedure for the monitor there are now two points on VS (y axis) corresponding to the two resistances entered. These two points establish a straight line L on a logarithm graph with VS varying in a logarithmic relationship with the return resistor of the patient circuit RP. This line L becomes the transfer function F(R) for resistance in and voltage out so that for a measured resistance RP there will be a corresponding VS. This line L has a slope m (gain) and an offset b where the line L intercepts the y axis which in the curve shown in Figure 10 is at VS = 1. The equation for line L is VS = F(RP) = mRP + b. The runmode for the electrosurgical apparatus then uses this equation in measurements of the resistance of the return part of the patient circuit. In practice, if the high and low values connected are not acceptable an audible sound will be emitted from a speaker or the like connected to U3 and further a bar graph connected to U3 may be used to instruct the user as to which resistance value to connect for monitor calibration.

By way of illustration only and not by limitation, there are listed below devices that have been found suitable for use in the illustrated circuits:

| Reference No. | Part No. |
|---|---|
| U1 | 87C64 INTEL |
| U2 | X2001 XICOR |
| U3 | 8031 INTEL |
| U4 | 74LS14 NAT SEMI |
| U5 | 74LS00 NAT SEMI |
| U7 | 74LSEZ NAT SEMI |
| U23 | DS1232 NAT SEMI |
| U21 | LM358 NAT SEMI |
| U31 | AD558 ANALOG DEVICES |
| U41 | LM339 NATL. SEMI |

Although the present invention has been described with a certain degree of particularity, it is understood that the present disclosure has been made by way of example and that changes in details of structure may be made without departing from the spirit thereof.

**Claims**

1. A method of calibrating selected operating characteristics of electrosurgical apparatus, comprising the step of storing selected calibration parameters establishing corresponding selected operating characteristics of electrosurgical apparatus, characterised in that the parameters are stored in a non-volatile, read-write storage device (U2) capable of being altered in place by electrical means for controlling of the subsequent operation of said electrosurgical apparatus.

2. A method according to claim 1, characterised by the steps of measuring a selected operating characteristic of the electrosurgical apparatus and adjusting the selected operating characteristic until the level of the operating characteristic is substantially equal to a standard value, and storing adjusted operating characteristic operating value in said non-volatile, read-write storage device (U2) for controlling of the subsequent operation of the electrosurgical apparatus.

3. A method according to claim 1 or 2, characterised in that the operating characteristic is the RF power output.

4. A method according to claim 3, characterised in that the RF power output is adjusted until it equals a test power of known value.

5. A method according to claim 1 or 2, characterised in that the operating characteristic is the patient contact quality measuring means of a return electrode contact monitor (35).

6. A method according to claim 5, characterised in that said measuring means measures electrical resistance.

7. A method according to claim 6, characterised in that a first resistance value is connected to said monitor (35) and a key actuated to input said resistance value into the storage device (U2) and a second resistance value is then connected to said monitor (35) and a key actuated to input the value into the storage device (U2).

8. A method according to any preceding claim, characterised in that said storage device (U2) is controlled by a programmed microprocessor (U3) and a memory (U1) providing processing instructions for said microprocessor.

9. A method according to claim 9, of calibrating the RF power output from an electrosurgical apparatus including indicating means displaying power output, select means for changing the RF power output, and controllable signal generator means, said method characterised by the steps of providing processing instructions for the microprocessor (U3) and storing initial power output values and drive variable values in the memory, storing calibrated RF power output values and corresponding drive variable values after the power output has been calibrated in the non-volatile read-write storage device (U2), coupling the amplified RF power output to a test box (20) having a resistance load (27), measuring the power consumed by said resistance load (27), said microprocessor (U2) driving said non-volatile, read-write storage device with a test power output value and retrieving a corresponding drive variable value stored in said non-volatile storage device (U2), said drive variable value corresponding to a request for the generation of test power, adjusting the amplified RF power output until the power equals the power measurement of the test box (26), said microprocessor (U3) adjusting the power values in said storage device (U2) to store a new power output value and corresponding drive variable value for a range of power output values for the next electrosurgical operation, thereby calibrating the RF power output.

10. Apparatus for calibrating selected operating characteristics of electrosurgical apparatus comprising storage means for storing selected calibration parameters establishing corresponding operating characteristics in electrosurgical apparatus, characterised in that the storage means is a non-volatile read-write storage device (U2) capable of being altered in place by electrical means for controlling the operation of said electrosurgical apparatus.

11. Apparatus according to claim 10, characterised in that said operating characteristic is a return electrode contact monitor (35).

12. Apparatus according to claim 10, characterised by a microprocessor (U3) for performing a lookup transfer function based on two different values of resistance input into said storage device (U2).

13. Apparatus according to claim 10, characterised in that said operating characteristic is RF power output.

14. Apparatus according to claim 13, characterised in that said storage device (U2) is controlled by a microprocessor (U3) having a memory (U1) which microprocessor in turn controls a controllable signal generating means (14).

15. Apparatus according to claim 14, characterised in that said microprocessor (U3) performs a lookup transfer function based on the tables in said storage device (U2) to change output power values to a corresponding drive variable value.

16. Apparatus according to claim 14, characterised in that said storage device (U2) stores a tabular group of a preselected number of number values per mode corresponding to a range of power in selected watt increments.

17. Apparatus according to claim 16, characterised in that said preselected number is 17 corresponding to 0 -192 watts in 16 watt increments.

18. Apparatus according to claim 16, characterised in that said preselected number is 17 corresponding to 192 - 320 watts in 32 watt increments.

19. Apparatus according to claim 10, characterised by first means for storing a calibration parameter establishing a corresponding operation level for RF power output in the non-volatile read-write storage device (U2), second means for measuring the selected RF power output during the operation of the electrosurgical apparatus, and third means for adjusting the selected RF power output until the RF power output is substantially equal to a standard value to effect calibration of RF power output whereby the adjusted operating characteristic is stored in said non-volatile read-write storage device (U2) for controlling the subsequent RF power output of said electrosurgical apparatus.

20. Apparatus for calibrating the RF power output of electrosurgical apparatus including a display (13) for indicating RF power output and up/down select keys (22,23) for changing the RF power output and a RF amplifier (16) providing the RF power output that is coupled to a load resistor (27) of a known value and providing a display of test power, characterised by a controllable signal generating means (14,15) responsive to the setting of said select keys (22,23) and coupled to said RF amplifier (16) to regulate the power output, a non-volatile read-write storage device (U2) for storing calibration drive variables according to the operation of the up/down select keys (22,23) whereby upon the movement of the select keys until the power output equals the test power the power output is calibrated, and a programmed microprocessor (U3) coupled to a memory and to said storage device (U2) for controlling said signal generating means (14) according to the drive variable stored in said storage device (U2).

21. Apparatus for calibrating the RF power output of electrosurgical apparatus coupled to a test load having a visual display (13) of power consumed by a load resistance (27) of known value, characterised by a programmed microprocessor (U3), a memory (U1) coupled to said microprocessor (U3) providing the processing instructions for the microprocessor (U3) and storing initial desired power values and corresponding drive variable values, a waveform generator (14) coupled to said microprocessor (U3) and to an RF amplifier (16) for providing a waveform from the RF amplifier (16) of a selected duty cycle, a digital to analog converter (15) coupled to said microprocessor (U3) and to the RF amplifier (16) for controlling the amplitude of the waveform produced by the waveform generator (14), a non-volatile read-write storage device (U2) coupled to said microprocessor (U3) for storing and outputting to the microprocessor (U3) the calibrated RF power output values and corresponding drive variable values after the RF power output has been calibrated, a display (13) for indicating actual RF power output that is coupled to the microprocessor (U3), said display initially indicating a calibration point number in amps corresponding to actual RF power being output, up/down keys (22,23) on the display for changing the RF power output, said microprocessor (U3) receiving a test power output value corresponding to said calibration point number from said storage device (U2), said microprocessor (U3) driving said non-volatile read-write storage device (U2) with said test power value and retrieving a drive variable value from a table stored therein, said microprocessor (U3) providing an output to said converter (15) or waveform generator (14) which in turn causes said RF amplifier (16) to output a corresponding RF power output, said up/down keys (22,23) being adjusted until the display power substantially equals the power reading in the test load, said microprocessor (U3) adjusting the power value in the storage device (U2) according to the new setting to store a new power output value and corresponding drive variable value thereby calibrating the apparatus to deliver the new RF power output during subsequent operations.

FIG.1

FIG.2

CAL

# FIG.3

THE DISPLAY 13 INDICATES CAL POINT #X

U3 GETS A TEST POWER CORRESPONDING
TO CAL POINT #X FROM THE EPROM MEMORY U1

U3 DRIVES U2
TABLE WITH TEST POWER
AND RECEIVES A DRIVE VARIABLE

DRIVE VARIABLE VALUE CORRESPONDS TO DAC
OR WAVEFORM REQUIREMENT FOR
GENERATION OF TEST POWER

U3 OUTPUTS DRIVE VARIABLE VALUE TO DAC 15
OR WAVEFORM GENERATOR 14

TO FIG.3A

⬇FROM FIG.3

THE OUTPUTTED DRIVE VARIABLE VALUE
PRODUCES A CORRESPONDING RF POWER

THE USER ADJUSTS UP\DOWN KEYS UNTIL
DISPLAYED POWER 13 = TEST POWER 28

U3 ADJUSTS U2
DRIVE VARIABLE VALUE UP\DOWN

DONE THIS POINT

ADVANCE TO NEXT TEST POWER POINT X+1

U3 FREEZES U2
TABLE VALUES OF U2

FIG.3A

CAL DONE THIS MODE

DRIVE VARIABLE

DESIRED POWER (WATTS)

FIG.4

NON-VOLATILE
READ-WRITE
STORAGE
DEVICE TABLE

POWER →

F(X)

LOOKUP

→ DRIVE VARIABLE

## FIG.5

RUN/CAL SWITCH

+5ᵥ

R51
4.7K

S1

2  6  CAL
RST

4

1  NC  RUN

3  5

KEY
BOARD  —12

DISPLAY  —13

+5ᵥ

U23

1
PB

2  6
TD RST

5  POR

3
TOL  ST  7

## FIG.6A

TO FIG.6B ⇨

FIG. 6B

# FIG. 6C

U2

| A14 | 2 | A6 | I/O7 | 17 | AD7 |
| A13 | 3 | A5 | I/O6 | 16 | AD6 |
| A12 | 4 | A4 | I/O5 | 15 | AD5 |
| A11 | 5 | A3 | I/O4 | 14 | AD4 |
| A10 | 6 | A2 | I/O3 | 13 | AD3 |
| A9 | 7 | A1 | I/O2 | 11 | AD2 |
| A8 | 8 | A0 | I/O1 | 10 | AD1 |
|  |  |  | I/O0 | 9 | AD0 |

NVEN    9  U7
CAL    10      8    19  NE
                    21  WE
                    18  OE
                    20  CE

WR     12  U7
CAL    13      11

PSEN   9  U5
       10     8

RD

+5v

U1

1  VPP
27 PGM          O7  19  AD7
A13  26 NC(A13) O6  18  AD6
     2  A12     O5  17  AD5
A11  23 A11     O4  16  AD4
A10  21 A10     O3  15  AD3
A9   24 A9      O2  13  AD2
A8   25 A8      O1  12  AD1
AD7  3  A7      O0  11  AD0
AD6  4  A6
AD5  5  A5
AD4  6  A4
AD3  7  A3
AD2  8  A2
AD1  9  A1
AD0  10 A0
     22 OE
     20 CE

R1    Q1
POR-\/\/\-

CAL

NVEN   9  U7
CAL   10      8

NVEN

RD
WR
PSEN
ALE

◁ TO FIG. 6B

EP 0 336 742 A2

FIG.8

FIG.7

ENTRY

LOW RESISTENCE VALUE IS INSERTED AND
KEY S2 IS PRESSED

THE OUTPUT VALUE OF THE MONITOR 35 IS
STORED IN U2 AS THE LOW RESISTANCE VALUE

HIGH RESISTENCE VALUE IS INSERTED AND THE
KEY S2 IS PRESSED

THE OUTPUT VALUE OF THE MONITOR 35
STORED IN U2 AS THE HIGH RESISTANCE VALUE

FIG.9

EXIT

NON-VOLATILE
READ-WRITE
STORAGE DEVICE
TABLE

RESISTANCE RP → F(R)
LOOKUP → VOLTAGE VS

FIG.11

FIG.10

EP 0 336 742 A2